# EUROPEAN PATENT APPLICATION

(11) **EP 2 792 670 A1**
(43) Date of publication of application: **22.10.2014**
(21) Application number: 14156554.9
(22) Date of filing: 03.10.2006
(51) Int. Cl.: C07C 215/40, C07C 215/42, C07C 229/42, C07C 233/47, C07F 9/24

(54) **Positively charged water-soluble prodrugs of mustards and related compounds with very high skin penetration rates**

(62) Divisional of application: 06809490.3
(71) Applicant: Techfields Biochem Co. Ltd, Shanghai N/A 200444 (CN); Yu, Chongxi, Plainfield, Illinois 60585 (US)
(72) Inventor: Yu, Chongxi, Plainfield, IL 60585 (US); Xu, Lina, Shanghai N/A 200444 (CN)
(74) Representative: Viering, Jentschura & Partner Patent- und Rechtsanwälte

(57) **Abstract**

The novel positively charged pro-drugs of mustards and related compounds in the general formula (1) 'Structure 1' were designed and synthesized. The compounds of the general formula (1) 'Structure 1' indicated above can be prepared from mustards and related compounds, by reaction with suitable alcohols, thiols, or amines and coupling reagents, such as N, N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, onzotriazol-1-yl-oxy-tris (dimethylamino)phosphonium hexafluorophosphate, et al. The positively charged amino groups of these pro-drugs not only largely increases the solubility of the drugs in water, but also bonds to the negative charge on the phosphate head group of membranes and pushes the pro-drug into the cytosol. The results suggest that the pro-drugs diffuse through human skin ∼100 times faster than do mustards and related compounds. In plasma, more then 90% of these pro-drugs can change back to the parent drugs in a few minutes. The prodrugs can be used medicinally in treating any mustards and related compounds-treatable conditions in humans or animals. The prodrugs can be administered transdermally for any kind of medical treatments and avoid most of the side effects of mustards and related compounds. Controlled transdermal administration systems of the prodrug enables mustards and related compounds to reach constantly optimal therapeutic blood levels to increase effectiveness and reduce the side effects of mustards and related compounds. Another great benefit of transdermal administration of these pro-drugs is that administering medication, especially to children, will be much easier.

## Description

### Technical Field

The present invention relates to the preparations of positively charged and watersoluble pro-drugs of mustards and related compounds and their medicinal use in treating any mustards and related compounds-treatable conditions in humans or animals. More specifically, the present invention is to enable fast skin penetration of mustards and related compounds for reducing the side effects of these drugs in the treatment of cancers, psoriasis and other diseases.

### Background Art

Cancer is the second leading cause of death in the United States, accounting for almost 25% of deaths. Cancer chemotherapy has been under intensive development for the past 40 years, resulting in cures of certain types of disseminated cancers that previously were fatal.

There are very few differences between cancer and normal cells according to present knowledge. Almost every cancer drug destroys both cancer and normal cells, especially the rapidly dividing normal body cells, such as hair follicles, cells lining the gastrointestinal tract, and bone marrow cells involved in the immune defense system. The most common side effects of present chemotherapy are nausea, hair loss, and increased susceptibility to infection. In addition, there are many other side effects that cancer patients experience.

Mustards are alkylating agents. They are very reactive compounds that can react with DNA, RNA, and enzymes and then kill the cancer cells. Mustards are used for treatment of leukemias, breast, ovarian, and lung cancer. Springer, et al. designed and synthesized many nitrogen mustard compounds for the treatment of cancers (Springer, C.J. ea al. U.S. Pat. No. 6852755, U.S. Pat. No.6916949, and U.S. Pat. No.6005002). Denny, et al. discussed the synthesis of nitrobenzyl mustard quaternary salts and their use as hypoxia-selective cytotoxic agents (Denny, W.A. et al, U.S. Pat. No.5691371). Glazier described prodrugs of phosphoramide mustard, isophosphoramide mustard and analogs (Glazier, A. U.S. Pat. No.5659061). Farquhar designed and synthesized novel antitumor aldophosphamide analogs (Glazier, A. U.S. Pat. No.5091552).

### Disclosure of Invention

### Technical Problem

There are very few differences between cancer and normal cells according to present knowledge. Almost every cancer drug destroys both cancer and normal cells, especially the rapidly dividing normal body cells, such as hair follicles, cells lining the gastrointestinal tract, and bone marrow cells involved in the immune defense system. The most common side effects of present chemotherapy are nausea, hair loss, and increased susceptibility to infection. There are also many other side effects that cancer patients experience.

### Technical Solution

Kadow et al. described a novel method for the delivery of antitumor drugs to tumor cells by the administration of a tumor-selective antibody-beta-lactamase conjugate that binds to tumor cells (Kadow, J. et al. U.S. Pat. No. 5773435). One alternative method of administering cancer drugs is topical delivery. Topical cancer drug delivery has several advantages. This method helps to avoid directly hurting the gastro-intestinal tract by cancer drugs and inactivation of the drugs caused by first pass metabolism in the liver and gastro-intestinal tract. It can provide local delivery of appropriate concentrations of a drug to the intended site of action without systemic exposure. Topical drug delivery methods may use a much smaller amount of drugs than the amount used for the systemic method and thus reduce the side effects of cancer drugs.

This invention relates to the preparation of novel positively charged water-soluble pro-drugs of mustards and related compounds and their use medicinally. The pro-drugs of mustards and related compounds have the general formula (1) 'Structure 1' or general formula (2) 'Structure 2'. 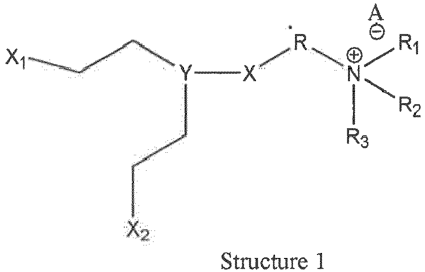 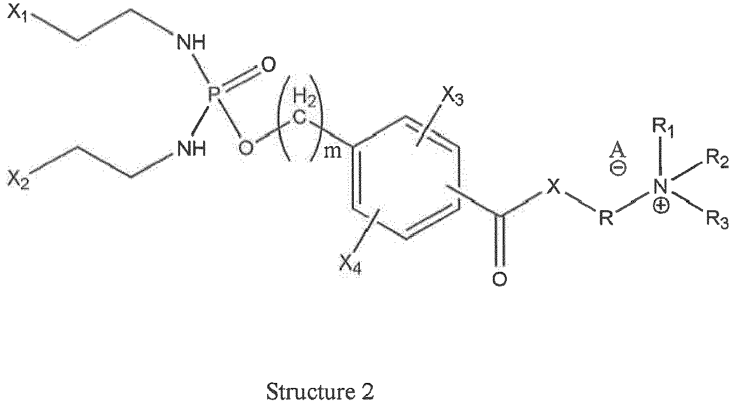 Wherein, R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, aryl or heteroaryl residues; A⁻ represents Cl⁻, Br⁻, F, I⁻, AcO⁻, citrate, or any negative ions; R represents a branched or straight chain, - (CH₂)ₙ-, wherein n=0, 1, 2, 3, 4, 5, 6, 7, 8, 9, 10 ......; X represents O, S, NH; X₁ represents Cl, Br, F, I, OSO₂R₄ (R₄ is lower alkyl, aryl, or heteroaryl groups), X₂ represents Cl, Br, F, I, OSO₂R₄ (R₄ is lower alkyl, aryl, or heteroaryl groups). X₃ and X ₄ represent H, F, Cl, Br, I, NO₂, CN, CF₃, NHCOCH₃, OCH₃, SCH₃, NH₂, NHCH₃, OCOCH₃,OCOC₂H₅, OC₂H₅, OC₃H₇, CH₃,C₂H₅, C₃H₇; m=0, 1, 2, 3, 4, 5, 6, 7, 8,......; All R, -(CH₂)ₙ - or -(CH₂)ₘ -groups are branched or straight chains and may include C, H, O, S, or N atoms and may have single, double, and triple bonds. Any CH₂ groups may be replaced with O, S, or NH. Y represents: 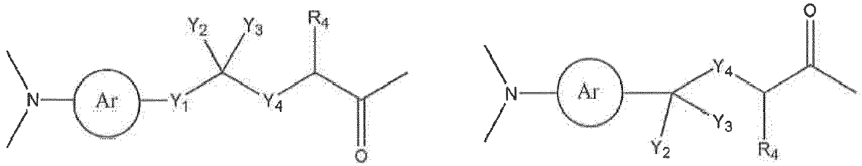 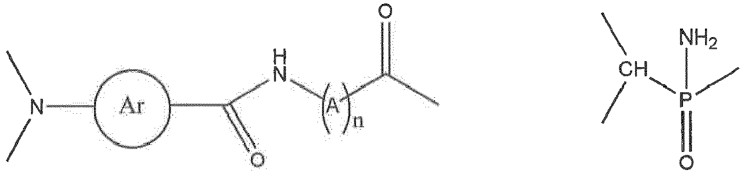 wherein Y₁ represents CH₂, O, S, NH; Y₂ and Y₃ taken alone are different and are H, OH, NHCOCH₃, NHCOC₂H₅, Cl, F, Br, I, or taken together are oxygen or 2 hydrogens; Y₄ represents CH₂, -(CH₂)ₙ -, O, S, NH; R₄ represents H, CH₂ CONH₂, CH₂ CH₂CONH₂, CH₂COOCH₃, CH₂ COOC₂H₅, CH₂NHCOCH₃, CH₂CH₂NHCOCH₃, CH₂ CH₂CH₂NHCOCH₃, CH₂CH₂CH₂CH₂NHCOCH₃, one of any alkyl, alkyloxyl, alkenyl or alkynyl residues having 1 to 12 carbon atoms, aryl or heteroaryl residues, A represents α-amino acid or β-amino acid residues, n=0, 1, 2, 3, 4, 5, 6, 7......; All R,-(CH₂)ₙ- or -(CH₂)ₘ -groups are branched or straight chains and may include C, H, O, S, or N atoms and may have single, double, and triple bonds. Any CH₂ groups may be replaced with O, S, or NH. 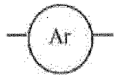 represents one of any ary or heteroaryl systems, they include, but are not limited to: 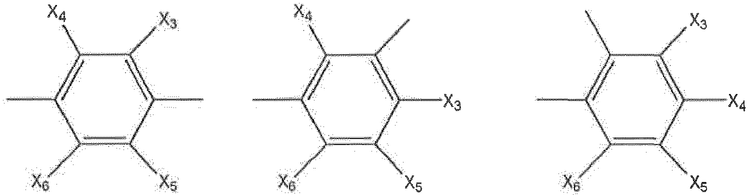 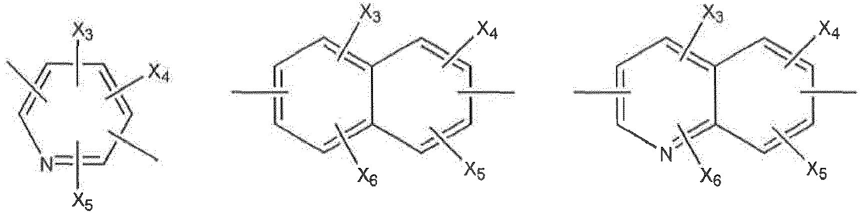 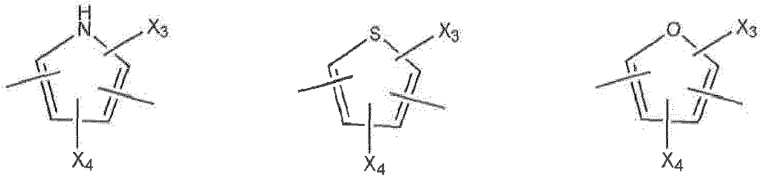 Wherein, X₃, X₄, X₅, and X₆ represent H, F, Cl, Br, I, NHCOCH₃, NO₂, CN, CF₃, OCH₃ , SCH₃, NH₂, NHCH₃, OCH₃, OC₂H₅, OC₃H₇, CH₃, C₂H₅, C₃H₇. When the bonds are not linked with atoms of the aryl or heteroaryl ring, that means that the bonds can be put into any position of the ring.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of membrane and very hydrophobic drugs will stay in the hydrophobic layer as part of the membrane due to their similarities and cannot enter the cytosol on the inside efficiently.

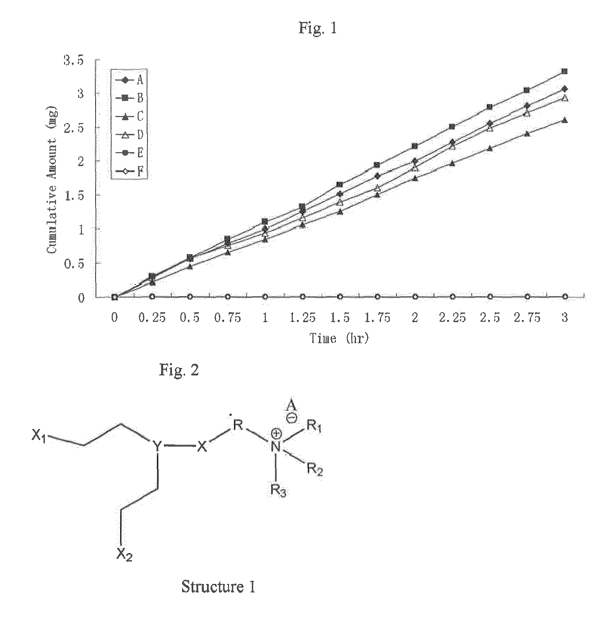

The goal of this invention is to make mustards and related compounds administrable transdermally (topical application) by increasing their solubility in the moisture on the skin surface and their penetration rate through the membrane and skin barrier to avoid hurting the GI tract. These novel pro-drugs of mustards and related compounds have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs in water. The solubility of theses pro-drugs of mustards and related compounds is >250 mg/ml and the solubility of mustards and related compounds is <0.1 mg/ml. In many instances, the lowest or rate-limiting step in the sequence is the dissolution of the drug. Mustards and related compounds have a very low solubility in the moisture on the skin surface and they will not pass across the barrier of skin in a molecular form efficiently. They stay outside of skin membranes for a long time and thus, will hurt the skin. When these new pro-drugs are administered transdermally in a dosage form such as a solution, spray, lotion, ointment, emulsion or gel, they will dissolve in the moisture of the skin surface immediately. The positive charge on the amino groups of these pro-drugs will bond to the negative charge on the phosphate head group of the membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. Due to the short stay outside of skin membranes and thus, the pro-drugs will not hurt the skin. The penetration rates of these prodrugs through human skin were measured in vitro by using modified Franz cells, which were isolated from human skin tissue (360-400 µm thick) of the anterior and posterior thigh areas. The receiving fluid consisted of 2 ml of 2% pH 7.4 phosphate buffer (0.2 M) and was stirred at 600 rpm. The cumulative amounts of these prodrugs and their parent drugs penetrating the skin versus time were determined by a specific high-performance liquid chromatography method. The results using a donor consisting of either a 20% solution of some of the prodrugs or a 20% suspension of some of the mustards and related compounds in 0.2mL of pH 7.4-phosphate buffer (0.2M) are shown in Figure 1. Apparent flux values of 1.01 mg, 1.10 mg, 0.85 mg, 0.94 mg, 0.01 mg, and 0.01 mg/cm²/h were calculated for N,N-diethylaminoethyl 4-[bis(2-chloroethyl)amino]benzenebutyrate.HBr, 4-[bis(2-chloroethyl)amino]-N-acetyl-L-phenylalanine N,N-diethylaminoethyl ester hydrobromide, N,N-bis(2-chloroethyl)aminophosphamide N,N-diethylaminoethyl ester hydrobromide, diethylaminoethyl 4-[bis(2-methylsulfonylethyl)amino]benzenebutyrate.HBr, chlorambucil, and melphalan diffuse through human skin. The results suggest that the pro-drugs diffuse through human skin -100 times faster than do mustards and related compounds. The results suggest that the positive charge on the dialkyaminoethyl group has a very important role in the passage of the drug across the membrane and skin barrier. Other prodrugs of the general formula (1) 'Structure 1' and general formula (2) 'Structure 2' have very high penetration rates and are very close to that of N,N-diethylaminoethyl 4-[bis(2-chloroethyl)amino]benzenebutyrate.HBr.

For evaluation of antitumor activity, a human myeloma cell line derived from the ascites of a patient with multiple myeloma was implanted into mice. The experiment was carried out on 11 groups of mice. Control group (A, orally), melphalan (B₁ and B₂, orally), chlorambucil (C₁ and C₂, orally), N,N-diethylaminoethyl 4-[bis(2-chloroethyl)amino]benzenebutyrate.HBr (D and D₂, transdermally), 4-[bis(2-chloroethyl)amino]-N-acetyl-L-phenylalanine N,N-diethylaminoethyl ester hydrobromide (E₁ and E₂, transdermally), and diethylaminoethyl 4-[bis(2-methylsulfonylethyl)amino]benzenebutyrate.HCl (F and F₂, transdermally). The body weight loss of mice was determined on day 21. The results are shown in table 1.

**Table 1: Extension of survival period and weight loss of cancer mice by use of mustards and their novel prodrugs.**

| Compounds | Dose (mg/kg) perday | Numbers of Mice | Survival Period (days) | Life Elongation Rate(%) | None Disease Rate | Weight Loss (%) |
|---|---|---|---|---|---|---|
| Control (A) | - | 7 | 45.5±1.6 | 100 | 0/7 | 1% |
| B₁ | 1.5 | 7 | 55.7±1.3 | 122 | 0/7 | 10% |
| B₂ | 3.0 | 7 | 88.5±1.8 | 195 | 2/7 | 18% |
| C₁ | 1.5 | 7 | 57.8±1.5 | 127 | 0/7 | 9% |
| C₂ | 3.0 | 7 | 90.2±1.9 | 198 | 3/7 | 17% |
| D₁ | 1.5 | 7 | 128.5±1. 3 | 282 | 4/7 | 5% |
| D₂ | 3.0 | 7 | 115.2±2. 1 | 253 | 4/7 | 10% |
| E₁ | 1.5 | 7 | 130.5±1. 6 | 287 | 4/7 | 4% |
| E₂ | 3.0 | 7 | 121.2±1. 8 | 266 | 3/7 | 9% |
| F₁ | 1.5 | 7 | 122.5±1. 7 | 269 | 4/7 | 6% |
| F₂ | 3.0 | 7 | 111.2±1. 9 | 244 | 3/7 | 11% |

The results show that the prodrugs demonstrated strong antitumor activity at 1.5 mg/kg dose and caused much less side effects (less weight loss) when they were administered transdermally.

All mustards that we used to make the prodrugs are known compounds. They are commercially available or can be made according to literature. The prodrugs of the general formula (1) 'Structure 1' and general formula (2) 'Structure 2' indicated above can be prepared from mustards and related compounds, by reaction with compounds of the general formula (3) 'Structure 3' by using coupling reagents, such as N,N'-Dicyclohexylcarbodiimide and N, N'-Diisopropylcarbodiimide, et al. 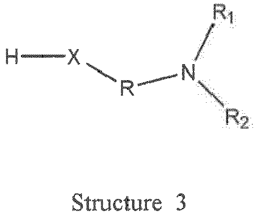 Wherein, R represents a branched or straight chain, -(CH₂)ₙ-, and n=0, 1, 2, 3, 4, 5, 6, 7, 8, 9, 10 .......; R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; X represents O, S or NH; and n=0, 1, 2, 3, 4, 5, 6, 7, 8, 9, 10......

When X represents O, the compounds of the general formula (1) 'Structure 1' and general formula (2) 'Structure 2' indicated above can be prepared from metal salts, organic base salts, or immobilized base salts of mustards and related compounds, by reaction with compounds of the general formula (4) 'Structure 4'. 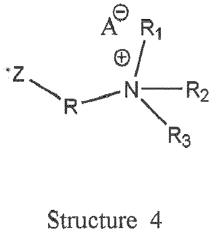 Wherein, R represents a branched or straight chain, -(CH₂)ₙ-, and n=0, 1, 2, 3, 4, 5, 6, 7, 8, 9, 10 ......; R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; R₃ represents H, one of any alkyl, alkyloxy, alkenyl, or alkynyl residues having 1 to 12 carbon atoms, aryl or heteroaryl residues; Z represents halogen, or p-toluenesulphonyl, A⁻ represents Cl⁻, Br⁻, F, r, AcO⁻, citrate, or any negative ions.

### Advantageous Effects

These pro-drugs of mustards and related compounds in the present invention have a lipophilic portion and a hydrophilic portion (the amine groups that exist in the protonated form at physiological pH). The positively charged amino groups of these pro-drugs have two major advantages. First, it largely increases the solubility of the drugs in water; when these new pro-drugs are administered transdermally in a dosage form such as a solution, spray, lotion, ointment, emulsion or gel, they will mix with moisture on the skin or other part of the body immediately. Second, the positive charge on the amino group of these pro-drugs will bond to the negative charge on the phosphate head group of the membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay on the skin or other part of the body, the pro-drugs will hurt the skin or other part of the body much less. Experiment results show that more than 90% of the pro-drugs were changed back to the parent drugs in a few minutes. The pro-drugs have a much better absorption rate and transdermal administration avoids the first pass metabolism, therefore the pro-drugs will have more strength than will mustards and related compounds at the same dosage. Another great benefit of transdermal administration of these pro-drugs is that administering medication, especially to children, will be much easier.

### Description of Drawings

Figure 1: Cumulative amounts of N,N-diethylaminoethyl 4-[bis(2-chloroethyl)amino]benzenebutyrate.HBr (A, 20% solution), 4-[bis(2-chloroethyl)amino]-N-acetyl- L-phenylalanine N,N-diethylaminoethyl ester hydrobromide (B, 20% solution), N,N-bis(2-chloroethyl)aminophosphamide N,N-diethylaminoethyl ester hydrobromide (C, 20% solution), diethylaminoethyl 4-[bis(2-methylsulfonylethyl)amino]benzenebutyrate.HBr (D, 20% solution), chlorambucil (E, 20% suspension), and melphalan (F, 20% suspension) crossing isolated human skin tissue in Franz cells (n=5). In each case, the vehicle was pH 7.4 phosphate buffer (0.2 M).
Figure 2. Wherein, R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, aryl or heteroaryl residues; A⁻ represents Cl⁻, Br⁻, F, I⁻, AcO⁻, citrate, or any negative ions; R represents a branched or straight chain,-(CH₂)ₙ-, and n=0, 1, 2, 3, 4, 5, 6, 7, 8, 9, 10 ......; X represents O, S, NH, X₁ represents Cl, Br, F, I, OSO₂R₄ (R₄ is lower alkyl, aryl, or heteroaryl groups), X₂ represents Cl, Br, F, I, OSO₂R₄ (R₄ is lower alkyl, aryl, or heteroaryl groups). See the Claim 1 for Y definition.

### Best Mode

### Preparation of N,N-diethylaminoethyl 4-[bis(2-chloroethyl)amino]benzenebutyrate.HBr

32.6 g (0.1 mol) of sodium 4-[bis(2-chloroethyl)amino]benzenebutyrate was dissolved in 100 ml of acetonitrile. 26 g (0.10 mol) of 2-Bromo-N,N-diethylethylamine.HBr in 50 ml of acetonitrile was added into the reaction mixture. The mixture was stirred for 3 h at RT. Solid is removed by filtration. The solvents were evaporated off. The solid product was collected by filtration and washed with ether. After drying, it yielded 35 g of the desired product (72.3%). Hygroscopic product; Solubility in water: 300 mg/ml; Elementary analysis: C₂₀H₃₃BrCl₂N₂ O₂; MW: 484.30. Calculated % C: 49.60, H: 6.87, Br: 16.50, N: 5.78, O: 6.61 ; Cl: 14.64; Found % C: 49.52, H: 6.89, Br: 16.55 N: 5.75, O: 6.65; Cl: 14.64. ¹H-NMR (400 MHz, D₂O): δ: 1.55 (t, 6H), 2.02(m, 2H), 2.27 (m, 2H), 2.54 (m, 2H), 3.23 (m, 4H), 3.51 (m, 2H), 3.60-3.65 (m, 8H), 4.51 (m, 2H), 6.55 (m, 2H), 6.95 (m, 2H).

### Mode for Invention

### Preparation of N,N-diethylaminoethyl 4-[bis(2-chloroethyl)amino] benzenebutyrate.HCl

30.4 g (0.1 mol) of 4-[bis(2-chloroethyl)amino]benzenebutanoic acid was dissolved in 300 ml of chloroform. 20.6 g of N, N'-Dicyclohexylcarbodiimide was added into the reaction mixture. 11.7 g of N,N-diethylaminoethanol and 0.2 g of 4-dimethylaminopyridine were added into the reaction mixture. The mixture was stirred overnight at 0°C. The solid was removed by filtration. The chloroform solution was washed with water (1 x 100 ml), 5% NaHCO₃ (1 x 100 ml) and water (3 x 100 ml). The organic solution was dried over anhydrous sodium sulfate. Sodium sulfate was removed by filtration. 4 g of HCl gas in methanol (10 ml) was added into the reaction mixture with stirring. Hexane (200 ml) was added. The solid product was collected by filtration. After drying, it yielded 35 g of the desired product (79.6 %). Hygroscopic product; Solubility in water: 300 mg/ml; Elementary analysis: C₂₀H₃₃Cl₃N ₂O₂; MW: 439.85. Calculated % C: 54.61, H: 7.56, N: 6.37; O: 7.27; Cl: 24.18; Found % C: 54.55; H: 7.58; N: 6.34, O: 7.29; Cl: 24.24. ¹H-NMR (400 MHz, D₂O): δ: 1.56 (t, 6H), 2.01(m, 2H), 2.25 (m, 2H), 2.55 (m, 2H), 3.22 (m, 4H), 3.52 (m, 2H), 3.60-3.65 (m, 8H), 4.50 (m, 2H), 6.55 (m, 2H), 6.95 (m, 2H).

### Preparation of 4-[bis(2-chloroethyl)amino]-N-acetyl-L-phenylalanine N,N-diethylaminoethyl ester hydrobromide

36.9 g (0.1 mol) of sodium 4-[bis(2-chloroethyl)amino]-N-acetyl-L-phenylalanine was dissolved in 100 ml of acetonitrile. 26 g (0.10 mol) of 2-Bromo-N,N-diethylethylamine.HBr in 100 ml of acetonitrile was added into the reaction mixture. The mixture was stirred for 3 h at RT. Solid is removed by filtration. The solvents were evaporated off. The solid product was collected by filtration and washed with ether. After drying, it yielded 38 g of the desired product (72.1 %). Hygroscopic product; Solubility in water: 300 mg/ml; Elementary analysis: C₂₁H₃₄BrCl₂N₂ O₂; MW: 527.32. Calculated % C: 47.83, H: 6.50, Br: 15.15, N: 7.97, O: 9.10, Cl: 13.45; Found % C: 47.77, H: 6.52, Br: 15.12 N: 7.96, O: 9.15; Cl: 13.48. ¹H-NMR (400 MHz, D₂O): δ: 1.54 (t, 6H), 2.02(s, 3H), 3.16 (m, 2H), 3.23 (m, 4H), 3.51 (m, 2H), 3.60-3.65 (m, 8H), 4.51 (m, 2H), 4.81 (m, 1H), 6.55 (m, 2H), 6.95 (m, 2H).

### Industrial Applicability

The pro-drugs of the general formula (1) 'Structure 1' and general formula (2) 'Structure 2' are superior to mustards and related compounds. They can be used medicinally in treating any mustards and related compounds-treatable conditions in humans or animals. They may be used for the treatment of skin cancer, breast cancer, oral cancer, colon-rectum cancer, respiratory system cancer, genital cancer, leukemia, or other cancers. They may also be used for the treatment of psoriasis.

### Sequence List Text

### Further Embodiments

[1] The compounds of the general formula (1)'Structure 1': 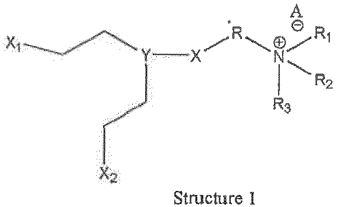 Wherein, R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkenyl residues having 1 to 12 carbon atoms, aryl or heteroaryl residues; A⁻ represents Cl⁻, Br⁻, F⁻, I⁻, AcO⁻, citrate, or any negative ions; R represents a branched or straight chain, - (CH₂)ₙ-, wherein n= 0, 1, 2, 3, 4, 5, 6, 7, 8, 9,10 ...... ; X represents O, S, NH; X₁ represents Cl, Br, F, I, OSO₂F₄ (R₄ is lower alkyl, aryl, or heteroaryl groups), X₂ represents Cl, Br, F, I, OSO₂R₄ (R₄ is lower alkyl, aryl, or heteroaryl groups). All R, -(CH₂)ₙ - or -(CH₂)ₘ -groups are branched or straight chains and may include C, H, O, S, or N atoms and may have single, double, and triple bonds. Any CH₂ groups may be replaced with O, S, or NH. Y represents: 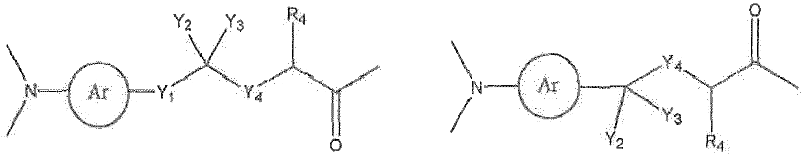 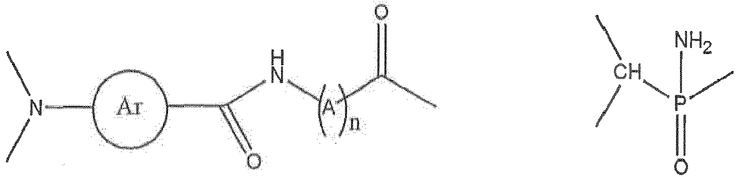 Wherein, Y₁ represents CH₂, O, S, NH; Y₂ and Y₃ taken alone are different and are H, OH, NHCOCH₃, NHCOC₂H₅, Cl⁻, F, Br, I, or taken together are oxygen or 2 hydrogens; Y₄ represents CH₂, -(CH₂)ₙ-, O, S, NH; R₄ represents H, CH₂CONH₂, CH₂CH₂CONH₂, CH₂COOCH₃, CH₂CNOOC₂H₅, CH₂NHCOCH₃, CH₂CH₂NHCOCH₃, CH₂CH₂CH₂NHCOCH₃, CH₂CH₂CH₂CH₂NHCOCH₃, one of any alkyl, alkyloxyl, alkenyl or alkynyl residues having 1 to 12 carbon atoms, aryl or heteroaryl residues, A represents α-amino acid or β-amino acid residues, n=0, 1, 2, 3, 4, 5, 6,7 ......; All R, -(CH₂)ₙ- or -(CH₂)ₘ-groups are branched or straight chains and may include C, H, O, S, or N atoms and may have single, double, and triple bonds. Any CH₂ groups may be replaced with O, S, or NH. 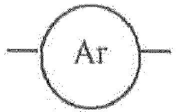 represents one of any ary or heteroaryl systems, they include, but are not limited to: 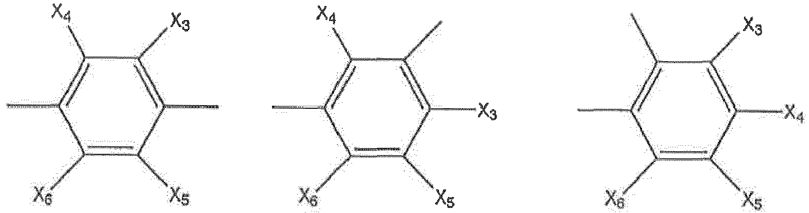 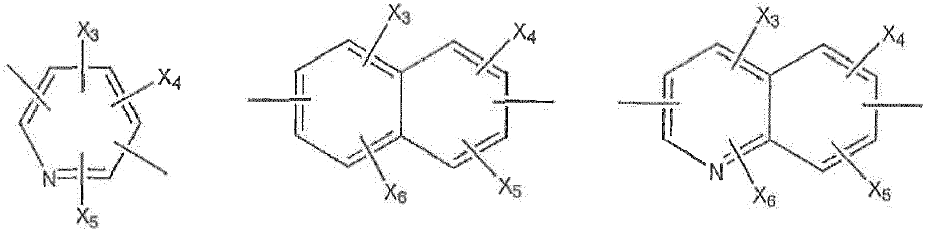 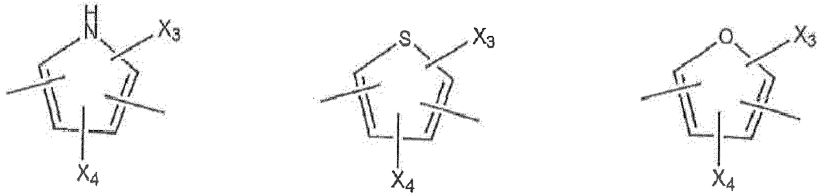 Wherein, X₃, X₄, X₅, and X₆ represent H, F, Cl, Br, I, NHCOCH₃, NO₂, CN, CF₃, OCH₃, SCH₃, NH₂, NHCH₃, OCH₃, OC₂H₅, OC₃H₇, CH₃, C₂H₅, C₃H₇. When the bonds are not linked with atoms of the aryl or heteroaryl ring, that means that the bonds can be put into any position of the ring.
[2] The compounds of the general formula (2) 'Structure 2'. 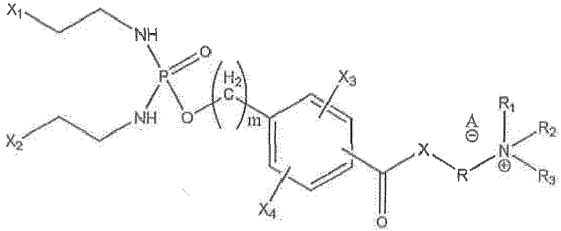 Wherein, R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, aryl or heteroaryl residues; A⁻ represents Cl⁻, Br⁻, F⁻, I⁻, AcO⁻, citrate, or any negative ions; R represents a branched or straight chain, - (CH₂)ₙ-, and n=0, 1, 2, 3, 4, 5, 6, 7, 8, 9, 10 ......., aryl or heteroaryl residues; X represents O, S, or NH; X₁ represents Cl, Br, F, I, OSO₂R₄ (R₄ is lower alkyl, aryl, or heteroaryl groups), X₂ represents Cl, Br, F, I, OSO₂R₄ (R₄ is lower alkyl, aryl, or heteroaryl groups). X₃ and X₄ represent H, F, Cl, Br, I, NO₂, CN, CF₃, NHCOCH₃, OCH₃, SCH₃, NH₂, NHCH₃, OCOCH₃, OCOC₂H₅, OC₂H₅, OC₃H₇, CH₃, C₂H₅, C₃H₇; m=0, 1, 2, 3, 4, 5, 6, 7, 8, ......; All R, -(CH₂)ₙ- or -(CH₂)ₘ- groups are branched or straight chains and may include C, H, O, S, or N atoms and may have single, double, and triple bonds. Any CH₂ groups may be replaced with O, S, or NH.
[3] Processes for the preparation of compounds of the general formula (1) 'Structure 1' and general formula (2) 'Structure 2' according to Embodiment 1 and Embodiment 2, wherein the compounds can be prepared from mustards and related compounds, by reaction with compounds of the general formula (3) 'Structure 3' by using coupling reagents, such as N,N'-Dicyclohexylcarbodiimide, N, N-Diisopropylcarbodiimide, O-(Benzotriazol-1-yl)-N,N,N,N-tetramethyluronium tetrafluoroborate, O-(Benzotriazol-1-yl)-N,N,N',N'-tetramethyluroniumhexafluorophosphate, Benzotriazol-1-yl-oxy-tris(dimethylamino)phosphoniumhexafluorophosphate, et al. 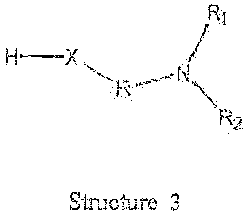 Wherein, R represents a branched or straight chain, -(CH₂)ₙ-, and n=0, 1, 2, 3, 4, 5, 6, 7, 8, 9,10 ...... ; R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; X represents O, S or NH.
[4] Processes for the preparation of compounds of the general formula (1) 'Structure 1' and general formula (2) 'Structure 2' according to Embodiment 1 and Embodiment 2, wherein the compounds can be prepared from metal salts, organic base salts , or immobilized base salts of mustards and related compounds, by reaction with compounds of the general formula (4) 'Structure 4'. 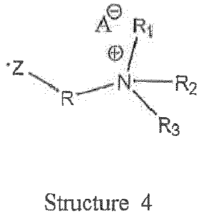 Wherein, R represents a branched or straight chain, -(CH₂)ₙ-, and n=0,1, 2, 3, 4,5, 6, 7, 8, 9, 10 ....; R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; R₃ represents H, one of any alkyl, alkyloxy, alkenyl, or alkynyl residues having 1 to 12 carbon atoms, aryl or heteroaryl residues; Z represents halogen, or p-toluenesulphonyl, A⁻ represents Cl⁻, Br⁻, F⁻, I⁻, AcO⁻, citrate, or any negative ions.
[5] Compounds of the general formula (1)'Structure 1' or general formula (2)'Structure 2' or a composition comprising of at least one compound of the general formula (1)'Structure 1' or general formula (2) 'Structure 2', as an active ingredient, according to Embodiment 1 and Embodiment 2, where they can be administered orally or transdermally (the latter is more preferable), for treating any mustards and related compounds-treatable conditions in humans or animals. The mustards and related compounds-treatable conditions include, but are not limited to skin cancer, breast cancer, oral cancer, colon-rectum cancer, respiratory system cancer, genital cancer, leukemia, or other cancers etc. They may also be used for the treatment of psoriasis.
[6] Transdermal therapeutic application systems of compounds of the general formula (1) 'Structure 1' or general formula (2) 'Structure 2' or a composition comprising of at least one compound of the general formula (1) 'Structure 1' or general formula (2) 'Structure 2', as an active ingredient, according to Embodiment 1 and Embodiment 2, for treating any mustards and related compounds-treatable conditions in humans or animals. The transdermal therapeutic application systems according to Embodiment 8, characterized in that these systems are a bandage or a patch comprising of one active substance-containing matrix layer and an impermeable backing layer. The most preferable system is an active substance reservoir, which has a permeable bottom facing the skin. By controlling the rate of release, this system enables the mustards and related compounds to reach constantly optimal therapeutic blood levels to increase effectiveness and reduce the side effects and mustards and related compounds.

## Claims

1. A compound selected from the group consisting of N,N-bis(2-chloroethyl)aminophosphamide N,N-diethylaminoethyl ester hydrobromide, 4-[bis(2-chloroethyl)amino] - N-acetyl-L-phenylalanine N,N-diethylaminoethyl ester hydrobromide, diethylaminoethyl 4-[bis(2-methylsulfonylethyl)amino]benzenebutyrate.HBr and a compound having a general formula of Structure 1 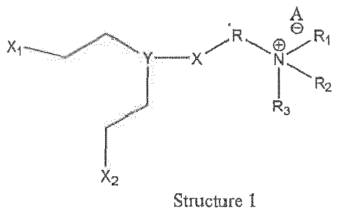 wherein
all R, -(CH₂)ₙ- or -(CH₂)ₘ- groups are branched or straight chains and may include C, H, O, S, or N atoms and may have single, double, and triple bonds; and any CH₂ groups may be replaced with O, S, or NH;
A⁻ is selected from any negative ions;
X is selected from the group consisting of O, S, and NH;
X₁ is selected from the group consisting of Cl, Br, F, I, and OSO₂R₄ (R₄ is aryl, or heteroaryl groups);
X₂ is selected from the group consisting of Cl, Br, F, I, and OSO₂R₄ (R₄ is aryl, or heteroaryl groups);
Y is selected from the group consisting of 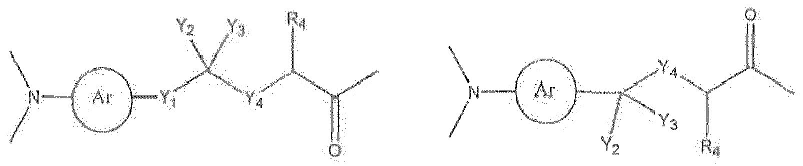 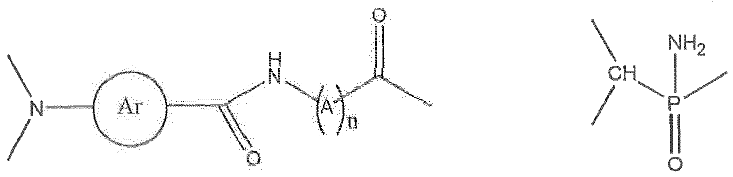
wherein
Y₁ is selected from the group consisting of CH₂, O, S, and NH;
Y₂ and Y₃ are independently selected from the group consisting of H, OH, NHCOCH₃, NHCOC₂H₅, Cl, F, Br, and I, or taken together are oxygen or 2 hydrogens;
Y₄ is selected from the group consisting of CH₂, -(CH₂)ₚ-, wherein p=0, 1, 2, 3, 4, 5, 6, or 7, O, S, and NH;
A is selected from the group consisting of α-amino acid and β-amino acid residues; 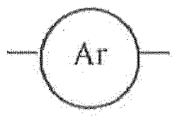 is selected from the group consisting of: 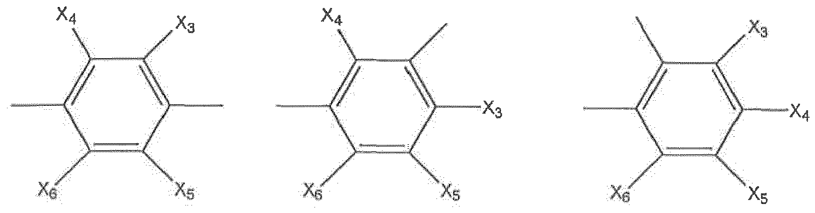 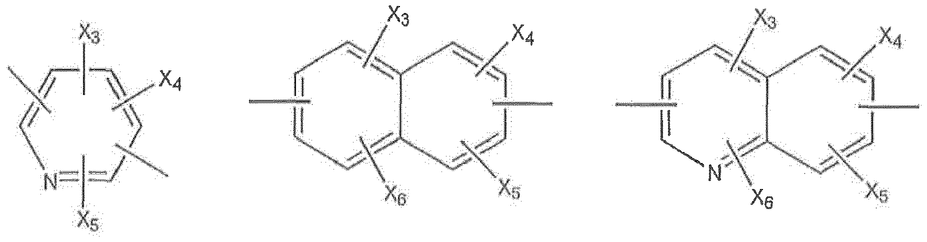 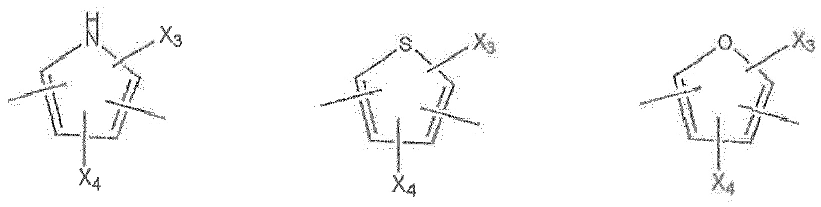 wherein X₃, X₄, X₅, and X₆ are independently selected from the group consisting of H, F, Cl⁻, Br, I, NHCOCH₃, NO₂, CN, CF₃, OCH₃, SCH₃, NH₂, NHCH₃, OCH₃, OC₂H₅, OC₃H₇, CH₃, C₂H₅, and C₃H₇, wherein when the bonds are not linked with atoms of the aryl or heteroaryl ring, that means that the bonds can be put into any position of the ring.

2. A compound having a general formula of Structure 2 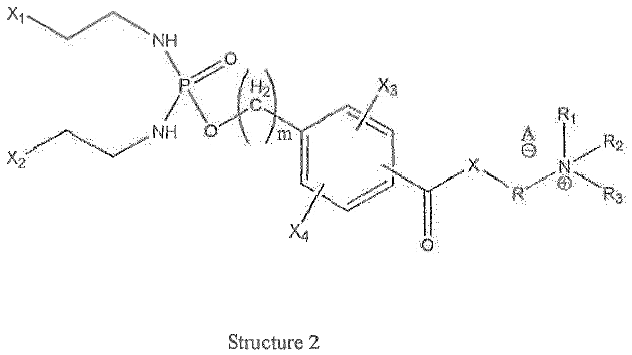 wherein
all R, -(CH₂)ₙ- or -(CH₂)ₘ- groups are branched or straight chains and may include C, H, O, S, or N atoms and may have single, double, and triple bonds; and any CH₂ groups may be replaced with O, S, or NH;
A⁻ is selected from any negative ions;
X is selected from the group consisting of O, S, and NH;
X₁ is selected from the group consisting of Cl, Br, F, I, and OSO₂R₄ (R₄ is aryl, or heteroaryl groups);
X₂ is selected from the group consisting of Cl, Br, F, I, and OSO₂R₄ (R₄ is aryl, or heteroaryl groups);
X₃ and X₄ are independently selected from the group consisting of H, F, Cl, Br, I, NO₂, CN, CF₃, NHCOCH₃, OCH₃, SCH₃, NH₂, NHCH₃, OCOCH₃, OCOC₂H₅, OC₂H₅, OC₃H₇, CH₃, C₂H₅, and C₃H₇; and
m = 0, 1, 2, 3, 4, 5, 6, 7, or 8.

3. The compound according to claim 1 or 2, wherein A⁻ is selected from the group consisting of Cl⁻, Br⁻, F⁻, I⁻ and AcO⁻.

4. A composition comprising one or more compounds according to any of claims 1 to 3.

5. The composition according to claim 4, further comprising water.

6. The compound according to any of claims 1 to 3, or the composition according to claim 4 or 5, for use in a method of treatment in human or animal, wherein the treated condition is treating a condition treatable by a mustard, and/or a related compound.

7. The compound or composition for use according to 6, wherein the treated condition is selected from the group consisting of the group consisting of skin cancer, breast cancer, oral cancer, colon-rectwn cancer, respiratory system cancer, genital cancer, leukemia, other cancers, and psoriasis.

8. The compound or composition for use according to claim 6 or 7, wherein the compound or composition is administered to deliver a therapeutically effective plasma level of the compounds or the composition.

9. The compound or composition for use according to any of claims 6 to 8, wherein the compound or composition is administered in the form of a solution, spray, lotion, ointment, emulsion or gel.

10. The compound or composition for use according to any of claims 6 to 9, wherein the compound or composition is administered orally or transdermally.

11. Transdermal therapeutic application systems comprising a compound according to any of claim 1 to 3, or a composition according to claim 4 or 5.

12. The transdermal therapeutic application systems according to claim 11, **characterized in that** the system is a bandage or a patch comprising an active-substance-containing matrix layer, and an impermeable backing layer.

13. The transdermal therapeutic application systems according to claim 11 or 12, **characterized by** having an active substance reservoir, which has a permeable bottom facing the skin.

14. The transdermal therapeutic application systems according to any of claims 11 to 13, **characterized by** a controlling means for controlling the rate of release, enabling the system to reach constantly optimal therapeutic blood levels.

15. The transdermal therapeutic application systems according to any of claims 11 to 14, for use in a method of treatment in human or animal, wherein the treated condition is treating a condition treatable by a mustard, and/or a related compound.
